# NEW EUROPEAN PATENT SPECIFICATION

(11) **EP 0 596 944 B2**
(45) Date of publication and mention of the opposition decision: **29.09.1999**
(45) Mention of the grant of the patent: 28.08.1996
(21) Application number: 92916093.5
(22) Date of filing: 28.07.1992
(51) Int. Cl.: A61F 13/15

(54) **ABSORBENT ARTICLE WITH IMPROVED ELASTICATED FLAPS**
ABSORPTIONSFAEHIGES PRODUKT MIT VERBESSERTEN ELASTISCH GEHALTENEN ANSAETZEN
ARTICLE ABSORBANT AVEC RABATS ELASTIQUES AMELIORES

(30) Priority: 30.07.1991 IT 910605
(43) Date of publication of application: 18.05.1994
(73) Proprietor: AZIENDE CHIMICHE RIUNITE ANGELINI FRANCESCO A.C.R.A.F. S.p.A., 00181 Roma (IT)
(72) Inventor: PALUMBO, Gianfranco, I-65125 Pescara (IT); DI GIROLAMO, Remo, I-65124 Pescara (IT)
(74) Representative: Bosotti, Luciano
(86) International application number: EP9201708
(87) International publication number: WO9302647

(56) References cited:
- EP-A- 0 207 904
- EP-A- 0 228 821
- EP-A- 0 243 013
- EP-A- 0 251 332
- EP-A- 0 346 477
- EP-A- 0 422 504
- WO-A-89/09549
- WO-A-90/14813
- DE-A- 3 038 535
- GB-A- 2 103 933
- US-A- 3 965 906
- US-A- 4 695 278
- US-A- 4 808 177

## Description

The present invention relates to disposable absorbent articles such as, for example, diapers for infants or incontinent adults and relates more specifically to absorbent articles of simplified structure having seal elements at the crotch with an improved ability to contain lateral leaks.

Disposable absorbent articles such as diapers for infants or incontinent adults, designed to absorb and retain body fluids, are well known and generally consist of an upper layer of non-woven fabric permeable to liquids and designed to be in contact with the user's skin, an impermeable plastics sheet and an element of absorbent material, commonly known as a pad, between the two said layers.

One problem linked to the use of such absorbent articles is the containment of leakage at the crotch through the lateral edges which, in use, are positioned round the user's legs.

The use along the lateral edges of the absorbent article of elastic elements designed to provide, in use, a seal round the user's legs, has been known for some time, as shown in United States patent US-A-3 860 003.

To improve the seal along the lateral edges, especially the containment of solid excrement which, by its nature, is not absorbed by the pad but tends to remain on the surface of the upper permeable layer, it has been proposed, as an alternative to or in combination with the conventional elastic elements along the lateral edges, to adopt elasticated flaps positioned inside the lateral edges of the absorbent article and either at the two sides of the pad or above it.

In use, the flaps are lifted from the surface of the absorbent article by the elastic elements incorporated in the said flaps, forming two longitudinal barriers able to guarantee better containment of lateral leakage.

For example, United States Patent US-A-4 704 116 describes a diaper of conventional structure with elasticated lateral edges and provided with two flaps positioned longitudinally, inwardly of these edges, above the pad, each flap in turn being delimited by two longitudinal edges and two ends.

Each flap is fixed to the upper layer of non-woven fabric along its outer longitudinal edge and at both ends, and is provided with at least one elastic element along the longitudinal edge facing the centre of the diaper.

The flaps are permeable to liquids and can either be formed by appropriately folding the upper non-woven fabric layer itself, or made separately and applied to the said upper layer.

This type of construction gives better protection against lateral leakage at the crotch but involves using more material to form the elasticated flaps.

In addition, as is commonly known in the art, if these flaps are to be impermeable to liquids, it is necessary for example that they be separately made of an impermeable material different from the material forming the upper permeable layer of the absorbent article; or, in the case of flaps made by folding the upper layer of non-woven fabric itself, it is necessary appropriately to treat the fabric or to add further impermeable elements.

A further development is presented in United States Patent US-A-4 808 177 which describes an absorbent article provided with the conventional elastics along the lateral edges to seal round the user's legs and with two so-called "floating" elasticated flaps positioned inwards of the said elasticated edges and characterized by a simplified structure.

In this patent each "floating" flap is made with an elastic element positioned longitudinally between the absorbent pad and the upper permeable non-woven fabric layer: the elastic element is fixed at both ends to the upper surface of the pad and to the lower surface of the non-woven fabric layer, while the intermediate portion remains unattaohed to the layers surrounding it.

In use the two elastic elements, contracting at the unattached intermediate portion, lift the upper layer at either side of the pad forming two longitudinal barriers.

Although this arrangement provides an absorbent article with simply constructed elasticated flaps, there is a need for an absorbent article with improved containment of lateral leakage characterised by impermeable elasticated flaps of simple structure whose manufacture does not involve a significantly larger use of raw material or the use of further treatment of materials compared to an absorbent article without elasticated flaps.

Also WO-A-89/09549 may be cited as a reference of some interest for the invention.

The object of the present invention is to improve the containment of lateral leakage of liquids and solid excrement in disposable absorbent articles.

According to the present invention, this object is achieved by providing an absorbent article specifically characterised by the features of Claim 1.

Additional characteristics and advantages of the invention will become apparent from the following description, given as a non-limitative example, with reference to the appended drawings, in which:
Figure 1 is a plan view of a disposable diaper according to the present invention;
Figure 2 is a sectional view along the axis BB' of the diaper of Figure 1 with the elasticated flaps lifted; and
Figure 3 is a plan view of a different configuration of a disposable diaper according to the present invention.

The absorbent articles of the present invention will be described here with reference to their use as disposable absorbent articles; these articles are worn by the users in direct contact with the body, for the purpose of absorbing body fluids, and are subsequently thrown away after a single use.

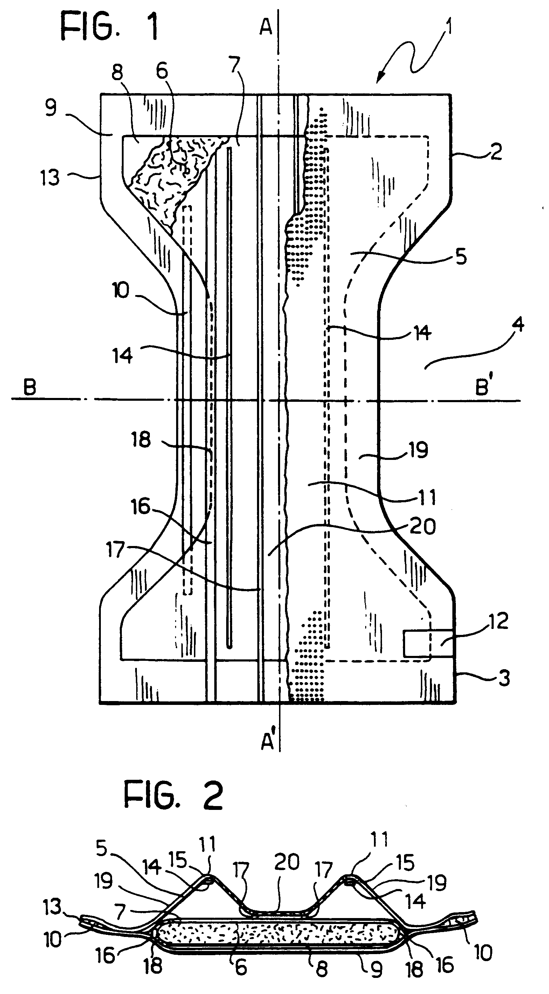

The disposable diaper shown in Figure 1 is a preferred embodiment of an absorbent article according to the present invention, but it is understood that the invention is applicable to other disposable absorbent articles, such as articles for the incontinent people and the like.

Figure 1 is a plan view of a diaper 1 spread flat, with a few portions of the structure sectioned to show the structure of the diaper more clearly; in particular the view shows the side of the diaper which comes into contact with the user.

Figure 1 shows two outermost regions, a front region 2 and a rear region 3, which in use are positioned round the user's waist, and a central region 4 between them which, in use, is positioned about at the crotch; it also shows a longitudinal axis AA' and a transverse axis BB'.

The diaper includes a continuous upper layer 5, provided for direct contact with the user's skin, an absorbent element 6 positioned between two tissue layers 7, 8, preferably of the wet-strength type, an impermeable plastics sheet 9, elastic elements 10 positioned at either side of the absorbent element 6 along the lateral edges of the diaper and provided to ensure, in use, a seal round the user's legs, and two elasticated flaps 11 made according to the present invention; also visible in the rear region 3 is one of the two adhesive tabs 12 commonly used to fix the diaper around the user's waist..

The continuous upper layer 5 and the plastics sheet 9 have the same shape and dimensions, following the outline 13 of the whole diaper.

The elasticated flaps 11, symmetrical relative to the axis AA', are positioned longitudinally along the lateral edges of the diaper, inwardly of the elastic elements 10; in the configuration illustrated the flaps 11 are parallel to the axis AA'.

Each flap 11 includes an elastic element 14 positioned under the continuous upper layer 5 and parallel to the longitudinal axis AA', which extends for substantially the entire length of the absorbent element 6.

The elastic elements 14 are fixed under tension along substantially the entire length of the inner surface of the continuous upper layers by fixing lines 15 formed for example by adhesive.

Each flap 11 also includes an outer seal line 16 and an inner seal line 17, both provided with adhesive for example, which join the continuous upper layer 5 to the underlying elements of the absorbent article and are positioned on either side of each elastic element 14, preferably parallel to this latter.

The outer and inner seal lines, 16 and 17, extend along the entire length of the diaper 1 and therefore together join between themselves the inner surface of the continuous upper layer 5 to the upper surface of the immediately underlying layer, which can be either the tissue layer 7 covering the absorbent element 6 or the impermeable plastics sheet 9.

The outer seal line 16 is positioned between the elastic element 10 along the lateral edge of the diaper and the elastic element 14 of the flap 11, for example between the elastic element 10 and the longitudinal edge 18 of the absorbent element 6, at least in the central region 4 of the diaper where the absorbent element 6 is characterised by a narrower width than at the front region 2 or rear region 3.

In the illustrated configuration the outer seal line 16 is positioned so that it is superimposed on the longitudinal edge 18 of the absorbent element 6 at the central region 4 of the diaper and is wide enough to ensure that along the said edge 18 the continuous upper layer 5 is fixed at the same time to the upper surface of the tissue layer 7 and to the impermeable plastics sheet 9.

The inner seal line 17 is positioned between the elastic element 14 of the flap 11 and the longitudinal axis AA' of the diaper 1; in particular the two elasticated flaps 11 can share a single inner seal line 17.

As shown in Figure 1, on the upper surface of the diaper 1, that is on the outer surface of the continuous upper layer 5, there are two lateral zones 19 outwardly of the two elastic elements 14 and a central zone 20 between the said two elastic elements 14.

In an entirely known way, in use the elastic elements 14 of the flaps 11 contract lifting the continuous upper layer 5 to form two raised barriers extending longitudinally along the sides of the absorbent element 6, as shown in Figure 2; the side of each barrier facing the lateral edge of the diaper is formed by the respective lateral zone 19 of the continuous upper layer 5, while the two sides facing the longitudinal axis AA' are formed by the central zone 20 of the said layer 5.

According to the present invention the continuous upper layer 5 which forms the two elasticated flaps 11 is made of a sheet of material which is permeable at the central zone 20 and impermeable at both lateral zones 19.

The use is particularly preferred of the covering structure for sanitary products described in Patent EP-B-0 207 904 held by the present applicant.

The said structure is perforated and includes an upper layer, designed to come into direct contact with the user's skin and made of a hydrophobic fibre non-woven fabric, an intermediate layer consisting of a plastics sheet, and a lower layer designed to face into the absorbent article and made of a hydrophobic fibre non-woven fabric; the three layers are joined to form a structure having a thickness between 200 microns and 700 microns.

According to the present invention, the continuous upper layer 5 of the diaper 1 consists of a covering structure of the type described that has been perforated, and therefore made liquid-permeable, only at the central zone 20, leaving the two lateral zones 19 imperforate and therefore impermeable.

Each flap 11 is therefore able, in use, to form a barrier characterised by a side facing the longitudinal axis AA' of the diaper 1 which is liquid permeable like all the central zone 20 of the continuous upper layer 5, and an impermeable side facing the lateral edge of the diaper 1, thereby obtaining improved containment of lateral leakage both of liquids and of solid excrement, with a simplified structure not requiring additional treatment or material.

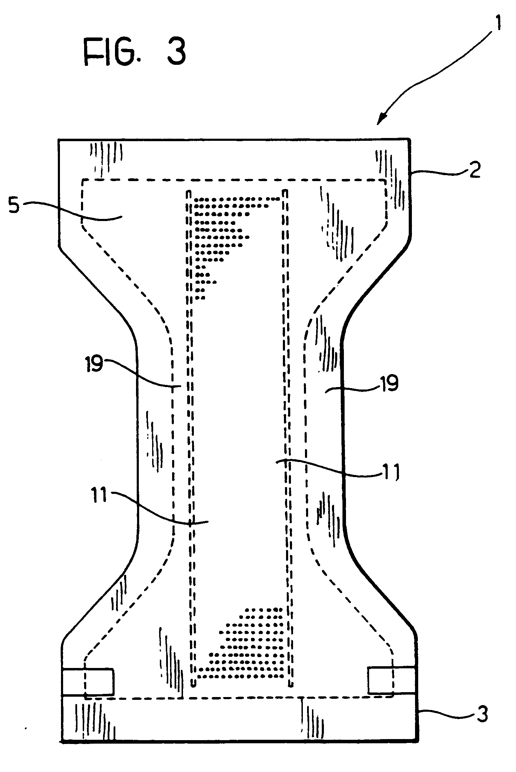

In an alternative configuration, shown in Figure 3, the diaper 1 with elasticated flaps 11 is characterised by a continuous upper layer 5 which is not perforated both at the two lateral zones 19 and at the front and rear regions 2, 3.

The two non-perforated zones at regions 2,3 act as barrier elements against leaks which, under certain conditions, can occur at the respective ends of the absorbent element 6 owing to flow-back of previously absorbed liquid.

This gives the further advantage of sealing the areas at the user's waist against moisture, using a structure characterised by the same manufacturing simplicity and without the need for additional elements such as, for example, the impermeable strips generally positioned under the upper permeable layer at the two ends of the diaper.

In a further alternative configuration, the elastic elements of the elasticated flaps are fixed for substantially the entire length to the inner surface of the continuous upper layer and at either end, situated at the front and rear end of the diaper respectively, are also fixed to the lower tissue layer covering the absorbent element, by fixing elements made, for example, of adhesive.

Naturally, the principle of the invention remaining the same. the details can be widely varied with respect to those described and illustrated without thereby departing from the scope of the present invention.

## Claims

1. An absorbent article including an upper layer (5), a lower liquid-impermeable layer (9) and an absorbent element (6) interposed between the upper layer (5) and the lower layer (9), the said upper layer (5) forming two elasticated flaps (11) extending alongside each other generally longitudinally of the absorbent article, characterised in that the upper layer (5) has a liquid-permeable central zone (20) between the two flaps (11) and two liquid-impermeable lateral zones (19) outwardly of the two flaps (11). and the two flaps (11) are elasticated by use of elastic elements (14) each one positioned straddling the central zone (20) and one of the lateral zones (19), and in that the upper layer (5) comprises
- an outer layer. designed to come into contact with the user's skin made of non-woven fabric constitued of hydrophobic fibres.
- an intermediate layer comprising a film of impermeable hydrophobic material, and
- an inner layer made of non-woven fabric constitued of hydrophobic fibres,
and in that the upper layer (5) is perforated through the three above layers in the central zone (20) but imperforate in the two lateral zones (19).

2. An article according to Claim 1, characterised in that the upper layer (5) is essentially continuous.

3. An article according to anyone of Claims 1 or 2 characterised in that it includes elastic parts (10) extending generally parallel to the two flaps (11) along the lateral edges of the absorbent element.

4. An article according to Claim 1, characterised in that the elastic elements (14) are glued to the upper layer 5 along most of their length.

5. An article according to Claim 1, characterized in that at least the outer layer of the upper layer (5), intended to come into contact with the user's skin, is treated with with hydrophilizing agents.

6. An article according to Claim 1 or Claim 4, characterized in that the elastic elements (14) each comprise only one elastic element formed by a rubber strip.

7. An article according to Claim 1 or Claim 4, characterised in that the elastic elements (14) each comprise two or more elastic members. preferably threads.

8. An article according to anyone of the preceding claims, characterised in that the upper layer (5) is impermeable at the longitudinal end zones of the article.

9. An absorbent article according to anyone of the preceding claims, in the form of a disposable diaper.

## Patentansprüche

1. Absorbierender Artikel umfassend eine obere Schicht (5), eine untere flüssigkeitsundurchlässige Schicht (9) und ein absorbierendes, zwischen der oberen Schicht (5) und der unteren Schicht (9) angeordnetes Element (6), wobei die obere Schicht (5) zwei elastisch ausgebildete, sich längs nebeneinander, im wesentlichen in Längsrichtung des absorbierenden Artikels erstreckende Streifen (11) bildet, dadurch gekennzeichnet, daß die obere Schicht (5) einen flüssigkeitsdurchlässigen mittleren Bereich (20) zwischen den beiden Streifen (11) und zwei flüssigkeitsundurchlässige seitliche Bereiche (19) außerhalb der beiden Streifen (11) aufweist, und daß die beiden Streifen (11) durch die Verwendung von elastischen Elementen (14) elastisch ausgebildet sind, wobei jedes den mittleren Bereich (20) und einen der seitlichen Bereiche (19) überspannend angeordnet ist, und
daß die obere Schicht (5) umfaßt:
- eine äußere Schicht, die aus Nonwoven bestehend aus hydrophoben Fasern hergestellt und für den Kontakt mit der Haut des Benützers vorgesehen ist,
- eine mittlere Schicht umfassend einen Film aus undurchlässigem hydrophobem Material, und
- eine innere Schicht hergestellt aus Nonwoven bestehend aus hydrophoben Fasern, und daß die obere Schicht (5) durch die drei oben angeführten Schichten hindurch im mittleren Bereich (20) perforiert ist, in den beiden seitlichen Bereichen (19) jedoch keine Perforierungen aufweist.

2. Artikel nach Anspruch 1, dadurch gekennzeichnet, daß die obere Schicht (5) im wesentlichen durchgehend ist.

3. Artikel nach einem der Ansprüche 1 oder 2, dadurch gekennzeichnet, daß er elastische Teile (10) umfaßt, die sich im wesentlichen parallel zu den beiden Streifen (11) entlang den seitlichen Rändern des absorbierenden Elementes erstrecken.

4. Artikel nach Anspruch 1, dadurch gekennzeichnet, daß die elastischen Elemente (14) mit der oberen Schicht (5,) entlang dem Großteil ihrer Länge verklebt sind.

5. Artikel nach Anspruch 1, dadurch gekennzeichnet, daß zumindest die äußere Schicht der oberen Schicht (5), welche für den Kontakt mit der Haut des Benützers vorgesehen ist, mit Hydrophilisierungsmitteln behandelt ist.

6. Artikel nach Anspruch 1 oder Anspruch 4, dadurch gekennzeichnet, daß die elastischen Elemente (14) jeweils nur ein aus einem Gummistreifen gebildetes elastisches Element umfassen.

7. Artikel nach Anspruch 1 oder Anspruch 4, dadurch gekennzeichnet, daß die elastischen Elemente (14) jeweils zwei oder mehr elastische Teile, vorzugsweise Fäden, umfassen.

8. Artikel nach einem der vorhergehenden Ansprüche, dadurch gekennzeichnet, daß die obere Schicht (5) in den längsverlaufenden Endbereichen des Artikels undurchlässig ist.

9. Absorbierender Artikel nach einem der vorhergehenden Ansprüche in Form einer Wegwerfwindel.

## Revendications

1. Article absorbant comprenant une couche supérieure (5), une couche inférieure imperméable aux liquides (9), et un élément absorbant (6) disposé entre la couche supérieure (5) et la couche inférieure (9), ladite couche supérieure (5) formant deux rabats élastiques (11) s'étendant le long l'un de l'autre généralement dans le sens de la longueur de l'article absorbant, caractérisé en ce que la couche supérieure (5) comporte une zone centrale perméable aux liquides (20) entre les deux rabats (11) et deux zones latérales imperméables aux liquides (19) vers l'extérieur des deux rabats (11), et les deux rabats (11) sont élastiques grâce à l'utilisation d'éléments élastiques (14), chacun à cheval sur la zone centrale (20) et une des zones latérales (19), et
en ce que la couche supérieure (5) comprend :
- une couche externe, destinée à contacter la peau de l'utilisateur, faite d'un non-tissé constitué de fibres hydrophobes,
- une couche intermédiaire comprenant un film de matériau hydrophobe imperméable, et
- une couche interne faite d'un non-tissé constitué de fibres hydrophobes,
et en ce que la couche supérieure (5) est perforée dans les trois couches ci-dessus dans la zone centrale (20) mais non perforée dans les deux zones latérales (19).

2. Article selon la revendication 1, caractérisé en ce que la couche supérieure (5) est sensiblement continue.

3. Article selon l'une quelconque des revendications 1 ou 2, caractérisé en ce qu'il comprend des éléments élastiques (10) s'étendant généralement parallèlement aux deux rabats (11) le long des bords latéraux de l'élément absorbant.

4. Article selon la revendication 1, caractérisé en ce que les éléments élastiques (14) sont collés à la couche supérieure (5) sur la majorité de leur longueur.

5. Article selon la revendication 1, caractérisé en ce qu'au moins la couche externe de la couche supérieure (5), destinée à contacter la peau de l'utilisateur, est traitée avec des agents qui rendent hydrophile.

6. Article selon la revendication 1 ou 4, caractérisé en ce que les éléments élastiques (14) comprennent chacun un élément élastique seulement formé par une bande de caoutchouc.

7. Article selon la revendication 1 ou 4, caractérisé en ce que les éléments élastiques (14) comprennent chacun deux éléments élastiques ou davantage, de préférence des fils.

8. Article selon l'une quelconque des revendications précédentes, caractérisé en ce que la couche supérieure (5) est imperméable aux zones d'extrémité longitudinales de l'article.

9. Article absorbant selon l'une quelconque des revendications précédentes, sous la forme d'une couche jetable.
